# EUROPEAN PATENT APPLICATION

(11) **EP 4 212 207 A1**
(43) Date of publication of application: **19.07.2023**
(21) Application number: 22151706.3
(22) Date of filing: 17.01.2022
(51) Int. Cl.: A61N 1/378, A61N 1/362

(54) **IMPLANTABLE MEDICAL DEVICE FOR EMITTING AN ELECTRICAL STIMULATION SIGNAL TO PERFORM A THERAPEUTIC ACTION**

(71) Applicant: BIOTRONIK SE & Co. KG, 12359 Berlin (DE)
(72) Inventor: Dörr, Thomas, 12437 Berlin (DE); Weiss, Ingo, 12435 Berlin (DE)
(74) Representative: Biotronik Corporate Services SE

(57) **Abstract**

An implantable medical device (3) for emitting an electrical stimulation signal to perform a therapeutic action comprises a processing circuitry (32) for generating said electrical stimulation signal, and an electrode pole arrangement (31) comprising a multiplicity of electrode poles (310-313) configured to emit said electrical stimulation signal to perform said therapeutic action. The processing circuitry (32) is configured to extract electrical energy from an energy signal (TS) received, using a first pair of electrode poles of said multiplicity of electrode poles (310-313), from an energy transmitting device (1) external to the implantable medical device (3) to obtain extracted electrical energy and to produce said electrical stimulation signal using said extracted electrical energy for emission by a second pair of electrode poles of said multiplicity of electrode poles (310-313).

## Description

The instant invention concerns an implantable medical device for emitting an electrical stimulation signal to perform a therapeutic action and a method for operating an implantable medical device.

An implantable medical device of this kind in particular comprises a processing circuitry for generating said electrical stimulation signal, and an electrode pole arrangement comprising a multiplicity of electrode poles configured to emit said electrical stimulation signal to perform said therapeutic action.

A conventional implantable medical device comprises an (electrochemical) battery serving to supply electrical energy for operation of the implantable medical device over a prolonged span of life, typically in the order of multiple months or even years. When the battery is depleted, the implantable medical device may have to be explanted for replacing the battery or for replacing the implantable medical device as a whole.

Approaches exist for employing passive implantable medical devices which do not comprise a battery, but are supplied with electrical energy using a separate transmitter device. An implantable medical device of this kind may for example be implanted intracardially, for example in the right or left ventricle or in the right or left atrium, in order to provide a therapeutic and/or diagnostic function within the patient's heart, wherein the transmitter is for example subcutaneously implanted and transmits an energy signal for reception by the implantable medical device for supplying energy for the device's operation.

There is a desire for providing an implantable medical device which may be easily operated in an implanted state, e.g. intracardially within the patient's heart for providing a painless therapeutic action within the heart, for example an antitachycardia pacing (in short ATP).

EP 2 769 750 A1 describes a device for use in providing stimulation to cardiac tissue, the device having a flexible, elongated body and an arrangement of electrode poles for emitting stimulation signals. Energy may be transferred to the device using for example an inductive coupling by sending energy to magnetic coils arranged within the device. Alternatively, energy for example may be supplied by acoustic radiation, for example by transmitting ultrasound waves.

WO 2012/013201 A1 describes an implantable electrode device, in particular for a cardiac pacemaker, the implantable electrode device being supplied with energy in an exclusively wireless manner via a time-variable magnetic field. The magnetic field is generated by an implanted control device.

US 2009/0018599 A1 describes an implantable cardiac tissue excitation system including an implantable pacing controller unit with a pulse generation circuit. The system also includes a lead with a lead body extending between a proximal lead end attachable to the patient control unit and a distal lead end configured to be implanted within the heart. Furthermore, the system includes a leadless electrode assembly configured to be implanted within the heart that includes a receiver to receive a wireless transmission, a charge storage unit to store charge energy, and an electrical stimulation circuit to deliver an electrical stimulus to cardiac tissue using the pacing control information and the charge energy.

It is an object of the instant invention to provide an implantable medical device and a method for operating an implantable medical device which allow in an easy and efficient way to provide for an intravascular therapy, in particular an intracardiac therapy, over a prolonged period of time.

In one aspect, an implantable medical device for emitting an electrical stimulation signal to perform a therapeutic action comprises a processing circuitry for generating said electrical stimulation signal, and an electrode pole arrangement comprising a multiplicity of electrode poles configured to emit said electrical stimulation signal to perform said therapeutic action. The processing circuitry is configured to extract electrical energy from an energy signal received, using a first pair of electrode poles of said multiplicity of electrode poles, from an energy transmitting device external to the implantable medical device to obtain extracted electrical energy and to produce said electrical stimulation signal using said extracted electrical energy for emission by a second pair of electrode poles of said multiplicity of electrode poles.

The implantable medical device serves for emitting an electrical stimulation signal to perform a therapeutic action. The implantable medical device in particular may be configured for an intracardiac implantation to provide e.g. a pacing action within the patient's heart. The implantable medical device specifically may be designed for a ventricular implantation in the right or left ventricle of the patient's heart or for an atrial implantation in the right or left atrium. Alternatively, the implantable medical device may be designed e.g. for an implantation in a coronary artery, e.g. of the left ventricle, of the patient's heart.

In particular, the implantable medical device may be configured for providing an antitachycardia pacing (ATP) within the patient's heart.

The implantable medical device may be designed for a complete intravascular implantation, such that the implantable medical device, in an implanted state, fully rests within a vessel or body cavity, for example within the patient's heart.

The implantable medical device may, in one embodiment, be a passive implantable medical device not comprising an electrochemical battery. The term "passive" herein denotes that the implantable medical device receives energy for operation solely from an energy transmitting device by means of an energy signal transmitted wirelessly by the energy transmitting device towards the implantable medical device. The implantable medical device hence may be implanted without an energy storage unit in the shape of a battery equipping the implantable medical device for a prolonged span of operation. Rather, energy for operation is extracted from an energy signal received from an energy transmitting device and is processed within the implantable medical device in order to generate and emit electrical stimulation signals at an implantation site for performing a desired therapeutic action, in particular an intracardiac pacing action.

The implantable medical device comprises a processing circuitry configured to extract electrical energy from an energy signal received by means of a first pair of electrode poles of the multiplicity of electrode poles. The electrode poles of the electrode pole arrangement serve to emit electrical stimulation signals to perform a therapeutic action and, likewise, to receive an energy signal in order to provide energy for operation of the implantable medical device. Hence, an arrangement of electrode poles of the implantable medical device is used for signal emission and for signal reception, the signal emission being for providing for a therapeutic action and the signal reception serving to receive energy to operate the implantable medical device.

The electrode pole arrangement comprises at least two electrode poles. The electrode pole arrangement for example may comprise three electrode poles or more than three electrode poles.

Herein, a first pair of electrode poles of the multiplicity of electrode poles is used to receive the energy signal as transmitted by the energy transmitting device. A second pair of electrode poles is used for emission of the electrical stimulation signal produced from extracted electrical energy as obtained from the energy signal. The first pair of electrode poles and the second pair of electrode poles, in one embodiment, have at least one electrode pole in common. The first pair of electrode poles and the second pair of electrode poles, in one embodiment, may use the same electrode poles. In another embodiment, if the multiplicity of electrode poles comprises at least three electrode poles, the first pair of electrode poles may for example use a first and a second electrode pole, whereas the second pair of electrode poles may use the first electrode pole and a third electrode pole different than the second electrode pole. In yet another embodiment, the two pairs may use completely different electrode poles.

In an implanted state, at least one of the electrode poles of the electrode pole arrangement or a multiplicity of the electrode poles generally are in contact with tissue within the patient. The pair of electrode poles for signal reception and the pair of electrode poles for signal emission herein may be designed and chosen to achieve a resulting body impedance as experienced by the respective pair of electrode poles. For example, the electrode poles of the first pair of electrode poles used for receiving the energy signal may be designed and chosen such that an effective body impedance for the first pair of electrode poles is smaller than 1000 Ohm, preferably smaller than 500 Ohm. The electrode poles of the second pair of electrode poles used for emission of the electrical stimulation signal may for example be designed and chosen such that an effective body impedance for the second pair of electrode poles is larger than 20 Ohm, preferably larger than 100 Ohm.

In one embodiment, the implantable medical device comprises a body on which the multiplicity of electrode poles is arranged. The body may for example be made from an electrically insulating material. For example, the body may be made from a biocompatible plastics material, a silicone material, a polyurethane (PU) material, a ceramics material, or a combination of such materials. Alternatively, the body of the implantable medical device may at least partially be made from a metal material, wherein the metal material comprises a coating at its outside such that an outer surface of the body of the implantable medical device is electrically non-conductive. The coating for example may be made from a parylene coating, a silicone coating, a polyurethane (PU) coating or the like.

The body may provide for a hermetically tied sealing of components, in particular the processing circuitry, enclosed within the body.

The body, for example on one end, may comprise a fixation device for fixing the body on tissue at an implantation site. The fixation device may for example be formed by a helical screw, by a stent-like structure or by an arrangement of fixation tines designed for engagement with tissue at the implantation site.

In one embodiment, the body may be flexibly bendable such that the body may be deformable in an implanted state and for example may adjust to a particular vessel shape at an implantation site. For providing such flexibility, the body may for example be formed from a silicone material.

In one embodiment, the body has an elongated shape generally extending along a longitudinal axis. For example, the body may have a length equal to or smaller than 10 cm and a cross-sectional diameter equal to or smaller than 10 mm. The body may comprise a circular cross section and may have a longitudinal, substantially cylindrical, tubular shape. Herein, at least some of the multiplicity of electrode poles of the electrode pole arrangement may be displaced with respect to one another along the longitudinal axis. Electrode poles hence are distributed along the longitudinal body of the implantable medical device to form spatially separated pairs of electrode poles for signal reception and/or transmission.

In one embodiment, at least some electrode poles of the multiplicity of electrode poles are formed by ring electrodes arranged on the body. The ring electrodes preferably are displaced with respect to one another along the longitudinal axis along which the body of the implantable medical device extends. The ring electrodes circularly extend about the body and are designed for signal transmission and/or reception. In particular, by means of the ring electrodes therapeutic stimulation signals may be emitted, and/or sense signals relating to e.g. cardiac activity may be sensed. In addition, the energy signal as transferred from the energy transmitting device may be received using the ring electrodes.

The energy signal is transferred from an energy transmitting device external to the implantable medical device. The energy transmitting device herein may be implanted, for example subcutaneously implanted, at an implantation site spatially separate from the implantation site of the implantable medical device. The energy transmitting device and the implantable medical device do not comprise a direct galvanic connection by means of a dedicated connection line, but energy is transmitted by the energy transmitting device to the implantable medical device in a wireless manner using the energy transferring (wireless) signal.

The energy transmitting device for example may be an implantable cardioverter defibrillator device. The implantable cardioverter defibrillator device may in particular be a non-transvenous implantable cardioverter defibrillator device (in short non-transvenous ICD), which is designed for implantation external to a patient's heart. In a non-transvenous implantable cardioverter defibrillator device, a generator device may for example be implanted subcutaneously in a patient. A lead, in a connected state, extends from the generator device, the lead being implanted such that it fully rests outside of the patient's heart. The lead may for example extend from the generator device towards a location in the region of the patient's sternum, the shock electrode hence being placed outside of the patient's heart for emitting an electrical shock pulse at a location external to the patient's heart.

The term "non-transvenous" in this respect in particular shall express that the lead of the non-transvenous implantable cardioverter defibrillator device does not extend transvenously into the heart, but fully rests outside of the patient's heart.

The implantable cardioverter defibrillator device generally is configured to emit a shock pulse for achieving a defibrillation. The implantable cardioverter defibrillator device may serve for monitoring and treating potentially life-threatening arrhythmias of a patient's heart. If the implantable cardioverter defibrillator device is a non-transvenous implantable cardioverter defibrillator device, the shock electrode in an implanted state of the defibrillator device is placed outside of the heart of the patient, for example in the region of the sternum of the patient, such that a shock pulse for achieving a defibrillation is generated outside of the heart. By employing the implantable cardioverter defibrillator device within a therapeutic system in combination with the implantable medical device, the implantable cardioverter defibrillator device operates in cooperation with the implantable medical device and in this way may be enabled to provide for a stimulation therapy, such as an intracardiac pacing therapy, for example an antitachycardia pacing. The implantable cardioverter defibrillator device herein transmits an energy signal to the implantable medical device, for example implanted intracardially within the right or left ventricle or within the right or left atrium, the energy signal being received by the implantable medical device and being processed to extract energy for producing electrical stimulation signals within the heart.

In another embodiment, the energy transmitting device may not have to be implanted, but is operated externally to the patient, for example by contacting the patient's body by the energy transmitting device.

In yet another embodiment, the energy signal may be coupled into the patient's body without physically contacting the patient's body, for example by using an electromagnetic induction technique for contactless energy transmission into the patient's body.

The energy signal in particular may be a signal at an elevated frequency, in particular in a predefined frequency range above 10 kHz, preferably about 30 kHz, for example at or around 100 kHz. The energy signal, in one embodiment, is at a frequency which does not cause a stimulation within the patient's body, in particular for body tissue in the region of the thorax.

The energy signal in particular may use a carrier signal at a predefined frequency, for example above 10 kHz, preferably about 30 kHz, for example at 100 kHz.

In one embodiment, the energy signal comprises a multiplicity of pulses formed by modulating the carrier signal. The pulses may be shaped to produce stimulation pulses at the implantable medical device for achieving a desired therapeutic action, such as a pacing action.

In one embodiment, the pulses may be modulated by the energy transmitting device to carry information. For example, the pulse width of the pulses and/or a repetition rate of the pulses may be modulated in order to provide for a signaling of information from the energy transmitting device to the implantable medical device. Operation of the implantable medical device may be controlled according to the signaling as transmitted from the energy transmitting device.

The pulse width of the pulses may in particular lie in a range between 0.05 ms and 10 ms. A voltage level of the pulses as received by the implantable medical device may for example lie in a range between 0.1 V and 5 V. A repetition interval between neighboring pulses may for example lie in a range between 20 ms and 2 s.

The energy signal may have a DC signal component, or does not have a DC signal component.

In one embodiment, the processing circuitry comprises a filter unit for performing a frequency filtering of the energy signal. The filter unit may for example be a bandpass filter which is designed to let a specific energy signal in a dedicated frequency range pass, but blocks signals outside of a passband. In this way energy may be specifically coupled into the implantable medical device, wherein different implantable medical devices may employ different frequency ranges and hence may be separately fed with energy by signal transmission from one or multiple energy transmitting devices. For example, multiple implantable medical devices may be implanted at different locations within the patient's heart in order to provide e.g. a multi-chamber stimulation within the patient's heart, wherein an energy transfer to the different medical devices may be conducted by using different energy signals at different frequency ranges.

In one embodiment, the processing circuitry comprises a demodulating circuit arrangement for demodulating the energy signal to obtain the extracted electrical energy. The demodulating circuit arrangement, in one embodiment, for example comprises an arrangement of one or multiple diodes, which serve to rectify the received energy signal and demodulate the signal in order to extract the electrical energy as carried by the modulated carrier signal.

In one embodiment, the processing circuitry may comprise a circuit arrangement for doubling or otherwise multiplying the voltage of the received signal for producing the stimulation signal.

In one embodiment, the processing circuitry comprises an energy storage circuit arrangement for storing the extracted electrical energy, the energy storage circuit arrangement comprising at least one capacitor. The at least one capacitor is charged by the extracted energy of the energy signal, such that operation of the implantable medical device for producing stimulation signals may be conducted by using energy as stored within the at least one capacitor.

The at least one capacitor of the energy storage circuit may, in cooperation with diodes of the demodulating circuit arrangement, be functional to provide for demodulation and energy extraction. By demodulating the energy signal the at least one capacitor is charged.

In one embodiment, an electrical stimulation signal may immediately, i.e. at the time of energy reception, be produced according to the charge of the capacitor, such that the implantable medical device substantially functions as a passive relay device. The therapeutic action hence takes place at the same time and is controlled by the transmission of the energy signal. The reception of the energy signal at the implantable medical device causes the production of a stimulation signal which is transmitted by means of the electrode pole arrangement. This may allow for a particularly easy construction of the implantable medical device, the implantable medical device not requiring any timing circuitry for timing stimulation signals. Rather, a control and timing may be conducted by the energy transmitting device, the energy transmitting device transferring energy to the implantable medical device for example in response to sense signals as sensed by the energy transmitting device, the implantable medical device receiving the energy and converting the energy into stimulation signals for providing a therapeutic action at the implantation site of the implantable medical device.

In another embodiment, the processing circuitry comprises a timing unit for timing a therapeutic action by producing a stimulation signal. In such embodiment, energy is transferred from the energy transmitting device to the implantable medical device, wherein the implantable medical device converts the energy and stores the energy in an energy storage arrangement, for example comprising one or multiple capacitors. Emission of stimulation signals however is controlled by the processing circuitry of the implantable medical device, for example according to a processing of sensed signals as sensed by the implantable medical device. The energy transmitting device hence serves to supply energy, wherein operation subsequently is carried out by the implantable medical device in a substantially autarkic manner by using the energy as received from the energy transmitting device to produce stimulation signals according to an internal control and timing at the implantable medical device.

Stimulation pulses of a stimulation signal as produced by the implantable medical device for performing a therapeutic action may for example have a pulse width in between 0.05 ms and 10 ms and may have a voltage level in between 0.1 V and 50 V. A repetition interval between pulses of the stimulation signal may for example lie in a range between 20 ms and 2 s.

If the implantable medical device serves as a passive relay device in that stimulation pulses are produced by immediate conversion of the energy signal as received from the energy transmitting device, the stimulation pulses may substantially correspond to demodulated pulses as obtained by demodulation from the energy signal and hence may exhibit substantially the same pulse width, voltage level and repetition interval as the pulses of the energy signal.

In one embodiment, the processing circuitry comprises a signal generation circuit arrangement for producing an electrical stimulation signal from the extracted electrical energy. The signal generation circuit arrangement may for example comprise an output circuit, for example in the shape of a so-called H bridge, allowing to produce stimulation signals in the shape of pulses having a particular polarity. The output circuit for example comprises a multiplicity of switches which may be switched in a pairwise manner to produce a therapeutic current path to produce a stimulation pulse of a particular polarity.

The signal generation circuit arrangement may for example be controlled by a timing circuitry.

In one embodiment, the signal generation circuit arrangement may comprise at least one current limiting device for limiting a current for producing the electrical stimulation signal. By means of the current limiting device, which for example may be a current limiting diode or a Zener diode, a current for producing a stimulation signal may be limited, for example to produce a stimulation pulse having a voltage level equal to or smaller than 50 V and a current level equal to or smaller than 100 mA.

The body of the implantable medical device may have a volume smaller than 5 cm³.

The implantable medical device may be implantable using a venous access.

The implantation may take place by using a catheter system, in particular within a minimal invasive procedure.

The implantable medical device may be explantable, for example using a catheter system.

The implantable medical device may comprise an RFID tag for identifying the implantable medical device. The RFID tag may in particular be readable in an implanted state of the implantable medical device.

The implantable medical device may be MRI compatible.

The implantable medical device in particular may be suitable to provide for a therapeutic action for providing for an antitachycardia pacing (ATP).

The implantable medical device may be configured for providing for a post-shock pacing, i.e., a pacing subsequent to a defibrillation action of a defibrillation device.

A system may include an implantable medical device as described above and an implantable cardioverter defibrillator device, in particular a non-transvenous implantable cardioverter defibrillator device for transferring the energy signal.

In another embodiment, a system may include an implantable medical device as described above and an external defibrillator for transferring the energy signal.

In yet another embodiment, a system may include an implantable medical device as described above and a life vest for transferring the energy signal.

In another aspect, a method for operating an implantable medical device for emitting an electrical stimulation signal to perform a therapeutic action comprises: generating, using a processing circuitry, said electrical stimulation signal; and emitting, using an electrode pole arrangement comprising a multiplicity of electrode poles, said electrical stimulation signal to perform said therapeutic action; wherein the processing circuitry, for generating said electrical stimulation signal, extracts electrical energy from an energy signal received, using a first pair of electrode poles of said multiplicity of electrode poles, from an energy transmitting device external to the implantable medical device to obtain extracted electrical energy and produces said electrical stimulation signal using said extracted electrical energy for emission by a second pair of electrode poles of said multiplicity of electrode poles.

The advantages and advantageous embodiments described above for the implantable medical device equally apply also to the method, such that it shall be referred to the above in this respect.

The idea of the invention shall subsequently be described in more detail with reference to the embodiments as shown in the drawings. Herein:
- Fig. 1: shows a schematic drawing of an implantable therapy system comprising an implantable medical device implanted intracardially and an implantable cardioverter defibrillator device in an implanted state in a patient;
- Fig. 2: shows a schematic drawing of an implantable medical device;
- Fig. 3: shows a schematic drawing of another embodiment of an implantable medical device;
- Fig. 4A: shows a schematic circuit diagram of a processing circuitry of an implantable medical device;
- Fig. 4B: shows a resulting stimulation pulse obtained by converting an energy signal pulse using the circuit of Fig. 4A;
- Fig. 5A: shows a schematic circuit diagram of another embodiment of a processing circuitry of an implantable medical device;
- Fig. 5B: shows a resulting stimulation pulse obtained by converting an energy signal pulse using the circuit of Fig. 5A;
- Fig. 6: shows a schematic circuit diagram of yet another embodiment of a processing circuitry of an implantable medical device;
- Fig. 7: shows a schematic circuit diagram of yet another embodiment of a processing circuitry of an implantable medical device;
- Fig. 8A: shows an example of an energy signal as transmitted by an energy transmitting device;
- Fig. 8B: shows a demodulated signal as obtained from the energy signal;
- Fig. 9: shows a schematic drawing of a processing circuitry of an implantable medical device; and
- Fig. 10: shows a schematic circuit diagram of an embodiment of a processing circuitry of an implantable medical device.

Subsequently, embodiments of the invention shall be described in detail with reference to the drawings. In the drawings, like reference numerals designate like structural elements.

It is to be noted that the embodiments are not limiting for the invention, but merely represent illustrative examples.

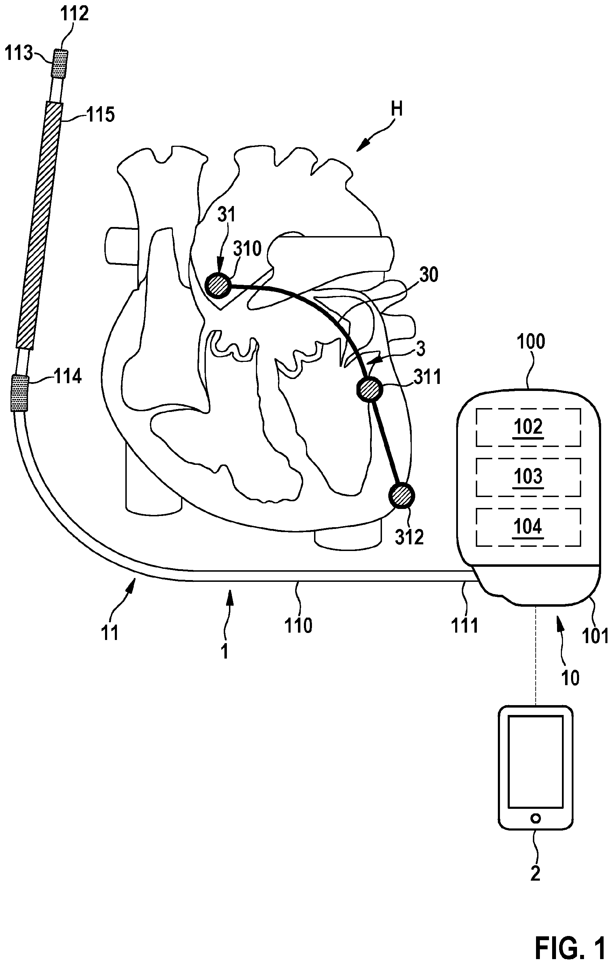

Referring to Fig. 1, in one embodiment a therapy system comprises an implantable medical device 3 and an implantable cardioverter defibrillator device 1 designed as a non-transvenous implantable cardioverter defibrillator device. The implantable medical device 3, in the shown setup, is implanted intracardially within the patient's heart H and is configured for providing a therapeutic action within the patient's heart H. The implantable medical device 3 herein is configured for receiving an energy signal from the implantable cardioverter defibrillator device 1, which serves as an energy transmitting device for the implantable medical device 3 and is implanted externally to the patient's heart H.

The non-transvenous implantable cardioverter defibrillator device 1, in the shown setup, is implanted such that the implantable cardioverter defibrillator device 1 is completely external to the heart H, the implantable cardioverter defibrillator device 1 comprising a generator device 10 encapsulated within a housing 100, and a lead 11 connected to the generator device 10 at a proximal end 111 and carrying electrode poles 113, 114 as well as a shock electrode 115 in the shape of a coil formed on a distal portion close to a distal end 112 of the lead 11. The electrode poles 113, 114, for example formed as ring electrodes on either side of the shock electrode 115, serve to sense cardiac signals for processing within the generator device 10 of the implantable cardioverter defibrillator device 1, such that based on sensed signals an arrhythmia may be identified and a shock pulse may be generated for providing for a defibrillation therapy.

The implantable cardioverter defibrillator device 1, in the embodiment of Fig. 1, is designed for a non-transvenous implantation, that is an implantation external to the patient's heart H. In particular, the lead 11 connected to the generator device 10 shall rest outside of the patient's heart H and shall not extend transvenously into the heart, the shock electrode 115 hence, in an implanted state, being placed outside of the heart H for providing for a defibrillation therapy.

For example, the generator device 10 may be implanted subcutaneously in a patient. The lead 11, with a lead body 110, may extend from the generator device 10 towards the sternum of the patient, the lead 11 for example tunneling through tissue in the region of the sternum and being placed beneath the sternum of the patient.

The generator device 10 generally comprises a processing circuitry 102 for controlling operation of the implantable cardioverter defibrillator device 1. In addition, the generator device 10 comprises a shock generation circuitry 103 and an energy storage 104, in particular in the shape of a battery. The processing circuitry 102 in particular serves to process signals sensed via a sensing arrangement formed by the electrode poles 113, 114 arranged on the lead 11 and additional poles, such as the shock electrode 115 and the housing 100 of the generator device 10.

The implantable cardioverter defibrillator device 1 may comprise communication circuitry for communicating with a device 2 external to the patient, for example within the context of a home-monitoring system.

By means of the implantable medical device 3 the implantable cardioverter defibrillator device 1 is enabled for providing a therapeutic function within the patient's heart H, in particular a therapeutic pacing function, for example for providing an antitachycardia pacing (ATP). The implantable cardioverter defibrillator device 1 herein is configured to transmit an energy signal to the implantable medical device 3 to supply the implantable medical device 3 with energy, for example using a pair of electrodes formed by the shock electrode 115 and the housing 100 of the generator device 10.

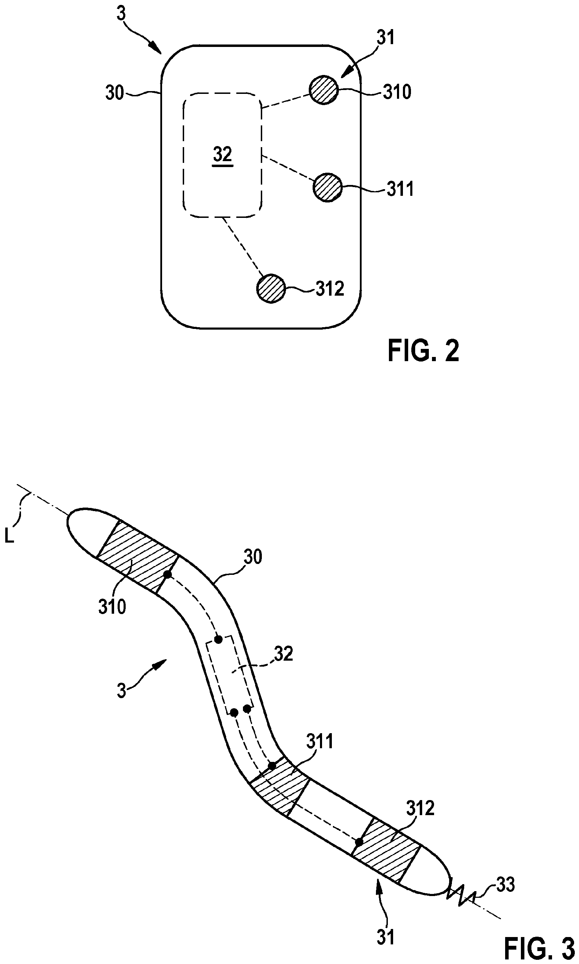

In the embodiment of Fig. 1, the implantable medical device 3 comprises a body 30 having an elongated shape and an electrode pole arrangement 31 comprising electrode poles 310, 311, 312 which are placed on the body 30 and are displaced with respect to one another on the body 30. The implantable medical device 3 may be a passive device which receives energy for operation from the implantable cardioverter defibrillator device 1 spatially separated from the implantable medical device 3. In one embodiment, the implantable medical device 3 does not comprise an active control device and an electrochemical battery, but serves to passively convert and relay energy received from the implantable cardioverter defibrillator device 1 to output an electrical stimulation signal within the patient's heart H. Referring now to Fig. 2, the implantable medical device 3 generally comprises a body 30 which encapsulates a processing circuitry 32 which is in operative connection with the electrode pole arrangement 31, namely the electrode poles 310, 311, 312. The processing circuitry 32 of the implantable medical device 3 serves to process an energy signal received from the implantable cardioverter defibrillator device 1 to extract energy from that signal and to convert the energy for producing stimulation pulses to be output by the electrode poles 310, 311, 312 of the electrode pole arrangement 31.

Referring now to Fig. 3, the body 30 may have an elongated shape extending longitudinally along a longitudinal axis L. The body 30 may for example be made from an electrically insulating material such as a biocompatible plastics material, a silicone, a polyurethane (PU) material, a ceramics material, or a combination thereof. In another embodiment, the body 30 may be at least partially made from a metal material and may comprise an electrically insulating coating, for example a parylene coating, a silicone coating, a polyurethane (PU) coating or the like, such that an outer surface of the body 30 is electrically non-conductive but provides for an electrical insulation towards the outside.

As visible from Fig. 3, the electrode poles 310, 311, 312 are displaced with respect to one another along the longitudinal axis L. The electrode poles 310, 311, 312 may for example be formed by ring electrodes circumferentially extending about the body 30.

The body 30 may have a circular cross section and may have an overall cylindrical, tubular shape. The body in particular may have a length smaller than 10 cm and a cross-sectional diameter smaller than 10 mm.

At one end, the implantable medical device 3 comprises a fixation device 33 for providing for a fixation on tissue, for example intracardiac tissue. The fixation device 33 may for example have the shape of a helical screw which may be screwed into tissue in order to provide for a fixation. In another embodiment the fixation device 33 may comprise one or multiple flexible tines, for example made from a shape-memory alloy material, such as nitinol. In yet another embodiment, the implantable medical device 3 may be held at an implantation site due to its shape, for example by providing a kink or bent within the body 30 in order to provide for a fixation in a coronary vessel or the like.

The body 30 may be flexibly bendable. In another embodiment, the body 30 is rigid.

The implantable medical device 3 with its body 30 may for example have a volume smaller than 5 cm³.

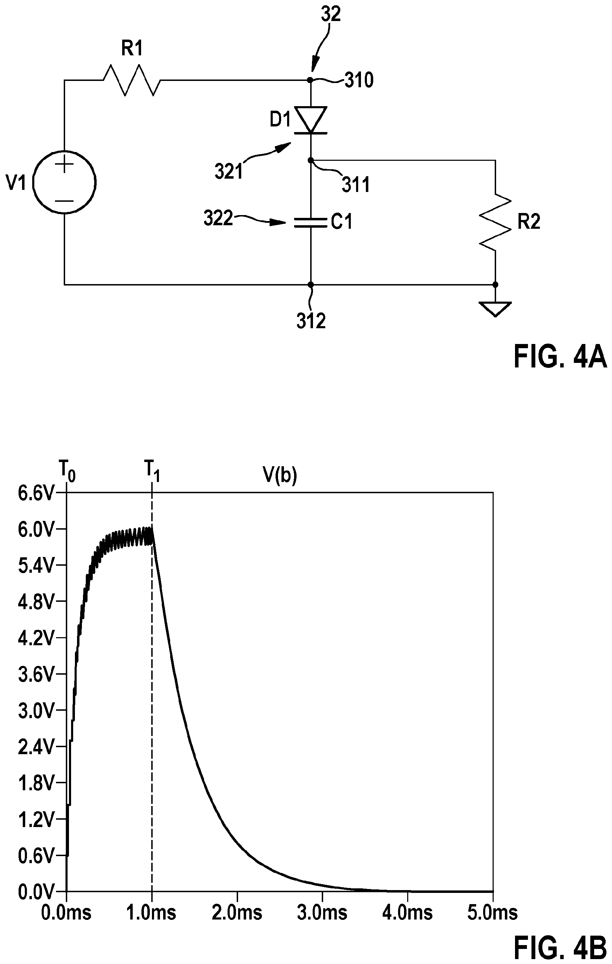

Referring now to Figs. 4A and 4B, in one embodiment the processing circuitry 32 is formed as a passive circuitry not requiring any energy supply arrangement such as an electrochemical battery. In the example of Fig. 4A, energy is received from an external energy transmitting device (in the embodiment of Fig. 1 the implantable cardioverter defibrillator device 1), which is modeled in the circuit diagram of Fig. 4A by the voltage source VI on the left. The energy signal is received via an effective body impedance, modeled in the circuit diagram of Fig. 4A by the resistance R1. The energy signal, in the embodiment of Fig. 4A, is received via a pair of electrode poles 310, 312, which feed the signal to a diode D1 and a capacitor C1 which are connected in series to form a combined demodulating circuit arrangement 321 and energy storage circuit arrangement 322.

By means of the diode D1 in combination with the capacitor C1 the signal received from the external energy transmitting device is converted and output via a pair of electrode poles 311, 312 connected to the capacitor C1. In this way a stimulation pulse is generated and output into body tissue, modeled in the circuit diagram of Fig. 4A by an effective body impedance R2.

Fig. 4B shows a resulting voltage signal across the capacitor C1 between the electrode poles 311, 312 upon receiving an input pulse signal across the electrode poles 310, 312. For the modeling, herein, an input voltage at a frequency of 32 kHz and an amplitude of 10 V at the voltage source VI is assumed. The effective body resistances R1, R2 are assumed to be 50 Ohm and 500 Ohm, respectively. The capacitor C1 has a value of 1 µF.

When supplying an energy signal in the shape of a voltage pulse modulated by a carrier frequency to the processing circuitry 32 substantially formed by the diode D1 in series with the capacitor C1, the energy signal is demodulated and converted into an output signal as shown in Fig. 4B. Namely, the capacitor C1 is charged and, while energy reception still goes on, is discharged across the electrode poles 311, 312 such that an electrical stimulation pulse is output across the electrode poles 311, 312 for performing a therapeutic action within the patient.

In the example of Fig. 4B, at time T0 the energy signal pulse starts, and energy reception at the processing circuitry 32 takes place via the electrode poles 310, 312. The voltage at the capacitor C1 builds up, and an electrical stimulation signal is output across the electrode poles 311, 312. At time T1 the energy signal pulse ends, upon which the capacitor C1 discharges, and the output voltage decays.

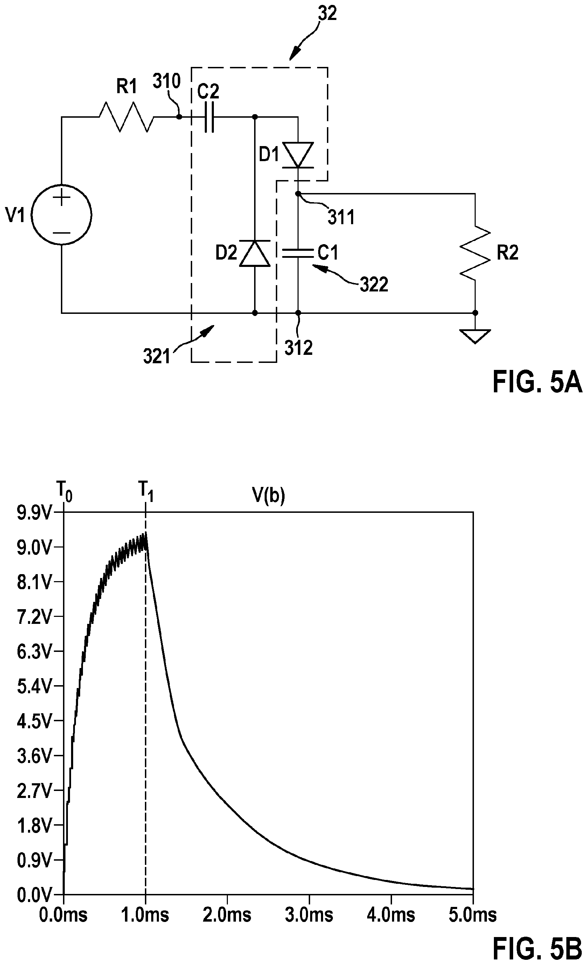

Referring now to Figs. 5A, 5B, in another example the processing circuitry 32 comprises diodes D1, D2 and capacitors C1, C2 which in combination form a demodulating circuit arrangement 321 and an energy storage circuit arrangement 322. An energy signal is received via the electrode poles 310, 312, the energy signal being modeled in the circuit diagram of Fig. 5A by the voltage source VI on the left. R1 models the effective body impedance for signal reception, and R2 the effective body impedance for signal transmission.

Fig. 5B shows the resulting signal waveform when receiving an energy signal pulse starting at time T0 and ending at time T1.

In the embodiments of Figs. 4A, 4B and 5A, 5B, the diode D1 respectively the diodes D1, D2 serve to rectify the received signal. In combination with the capacitor C1 respectively the capacitors C1, C2 the received signal is demodulated in that the carrier signal is removed to obtain the signal waveforms as indicated in Figs. 4B and 5B.

The carrier frequency for transmitting the energy signal by means of the energy transmitting device (in the embodiment of Fig. 1 the implantable cardioverter defibrillator device 1) uses a carrier frequency above 10 kHz, preferably about 30 kHz, for example around 100 kHz. The frequency of the carrier signal is chosen such that a tissue stimulation by transmission of the energy signal is avoided.

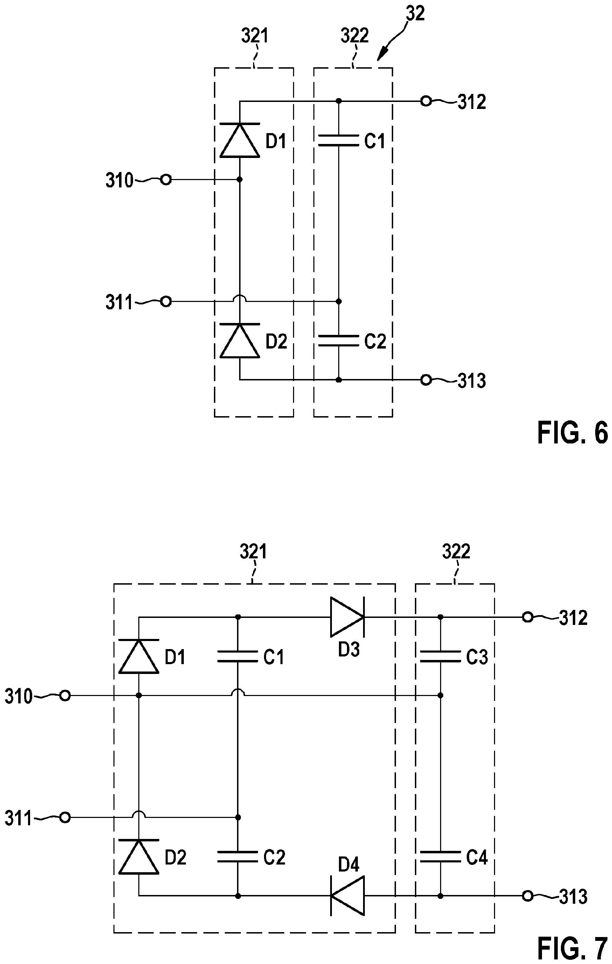

In another embodiment shown in Fig. 6, the processing circuitry 32 comprises diodes D1, D2 and capacitors C1, C2 which are arranged to form a voltage doubler, for example according to a so-called Delon circuit.

In yet another embodiment shown in Fig. 7, diodes D1 to D4 and capacitors C1 to C4 are arranged to form a voltage quadrupler.

In both embodiments of Fig. 6 and Fig. 7 the energy signal is received by a pair of electrode poles 310, 311, whereas the electrical stimulation signal is output using a pair of electrode poles 312, 313 across the capacitors C1, C2 (in the embodiment of Fig. 6) respectively across the capacitors C3, C4 (in the embodiment of Fig. 7). The receiving pair of electrode poles 310, 311 and the outputting pair of electrode poles 312, 313 hence do not have an electrode pole in common in the embodiments of Figs. 6 and 7.

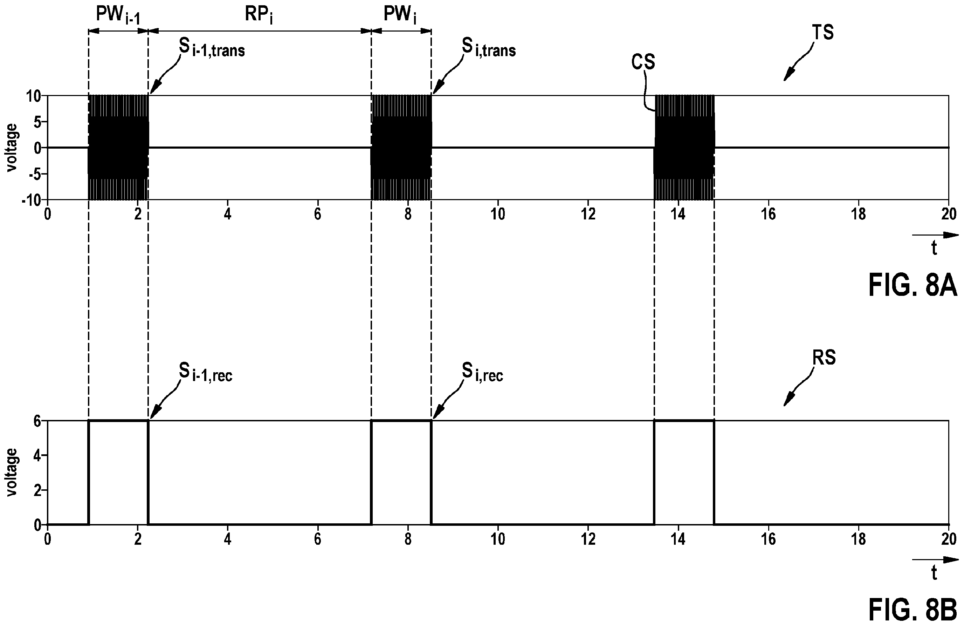

Referring now to Fig. 8A, in one embodiment the energy transmitting device (in the embodiment of Fig. 1 the implantable cardioverter defibrillator device 1) outputs an energy signal TS which comprises pulses S_{i-1,trans}, S_{i,trans} formed by modulation using a carrier signal CS at a defined frequency. The pulses may have an equal pulse width PWᵢ₋₁, PWᵢ, or may differ in their pulse width PWᵢ₋₁, PWᵢ. Likewise, a repetition rate RPᵢ in between neighboring pulses may be constant or may vary.

The pulse width PWᵢ₋₁, PWᵢ may for example lie in a range between 0.05 ms to 10 ms. The pulses S_{i-1,trans}, S_{i,trans} may have a voltage level in between 0.1 V to 50 V. The pulse repetition interval RPᵢ may lie in a range between 20 ms and 2 s.

Hence, the implantable medical device 3, using the processing circuitry 32, converts the received signal RS by demodulation such that, effectively, energy pulses S_{i-1,rec}, Si,rec are extracted from the received signal. In particular, by demodulation the carrier signal CS is removed, and the signal is rectified such that the signal energy is extracted for producing an electrical stimulation signal to be output using the electrode arrangement 31.

In the circuit diagrams as depicted in Figs. 4A, 4B, 5A, 5B, 6 and 7 it is assumed that the implantable medical device 3 functions as a passive device which does not comprise an active control circuitry, in particular a timing function, and an energy storage for a prolonged storage of electrical energy, such as an electrochemical battery. Rather, in the embodiments of Figs. 4A, 4B, 5A, 5B, 6 and 7 the received energy is directly converted to produce an output electrical stimulation signal for performing a therapeutic action at the implantation site of the implantable medical device 3. Therapeutic parameters herein are controlled by the signal transmission of the energy transmitting device.

This however is not restrictive for the instant invention. Rather, the implantable medical device 3 may comprise a timing function to control a therapeutic action in an autarkic manner. In such embodiments, therapeutic parameters are not controlled by the signal transmission of the external energy transmitting device (in the embodiment of Fig. 1 the implantable cardioverter defibrillator device 1), but by the implantable medical device 3 itself. In such embodiments, energy is received at the implantable medical device 3 from the external energy transmitting device, wherein the energy is converted and stored at the implantable medical device 3 and is used for operation of the implantable medical device 3.

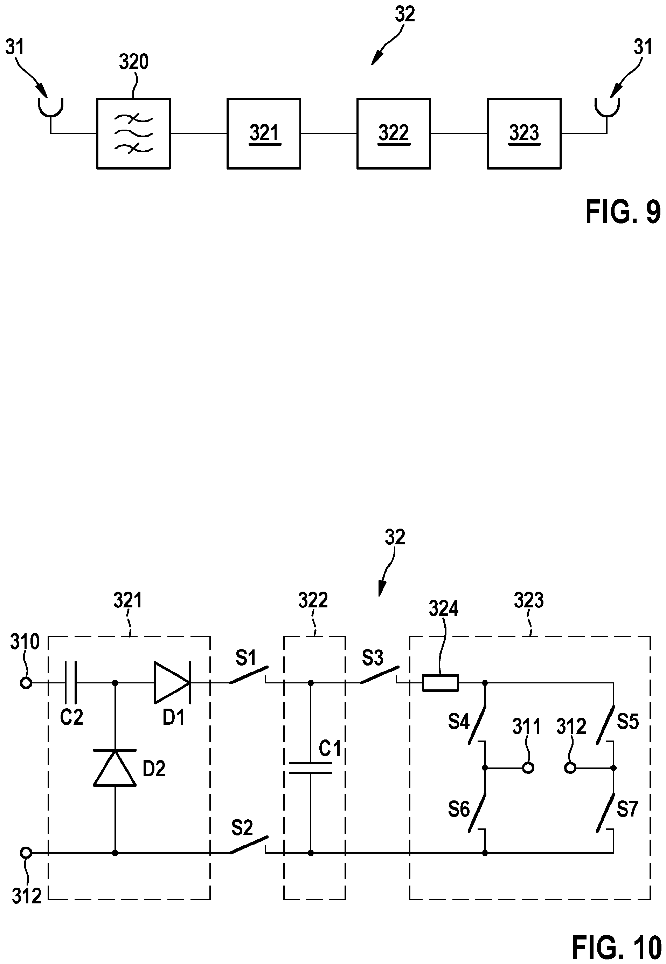

Referring now to Fig. 9, in a general embodiment of an implantable medical device 3 comprising advanced control circuitry, the processing circuitry 32 comprises a filter unit 320 for filtering an energy signal received via the electrode pole arrangement 31. The filter unit 320 may for example be a bandpass filter for passing a signal in a particular frequency range, but blocking signal components outside of that frequency range. By using such a filter unit 320, a particular energy signal may be received at the implantable medical device 3, wherein different implantable medical devices 3 may be configured to receive energy signals at different frequencies, hence allowing to use multiple implantable medical devices 3 in parallel, for example to achieve a multi-chamber stimulation within a patient's heart H. The filter unit 320 passes the received signal to a demodulating circuit arrangement 321 at which the signal is demodulated and fed to an energy storage circuit arrangement 322 at which energy extracted from the received signal is stored.

In a signal generation circuit arrangement 323, electrical stimulation signals are produced from the energy as stored in the energy storage circuit arrangement 322 and output by means of the electrode pole arrangement 31.

Referring now to Fig. 10, in a particular embodiment the demodulating circuit arrangement may comprise diodes D1, D2 and a capacitor C2, similar as in the embodiment of Fig. 5A, for receiving an energy signal using a pair of electrode poles 310, 312 and for demodulating the energy signal. The demodulating circuit arrangement 321 may be selectively coupled to the capacitor C1, which forms an energy storage circuit arrangement 322, by means of switches S1, S2. The energy storage circuit arrangement 322 of the capacitor C1 in turn may be selectively coupled, using a switch S3, to a signal generation circuit arrangement 323 comprising an arrangement of switches S4 to S7 forming an H bridge for producing electrical stimulation signals to be output via a pair of electrode poles 311, 312.

During operation, energy is received via the demodulating circuit arrangement 321 and is used for charging the capacitor C1 of the energy storage circuit arrangement 322 while the switches S1, S2 are in a closed position and the switch S3 is in an open position. When the capacitor C1 is charged, the switches S1, S2 may be opened. For producing an electrical stimulation signal, then, the switch S3 may be closed and, using the signal generation circuit arrangement 323, output pulses of a desired polarity may be generated by selectively closing a pair of switches S4, S7 respectively S5, S6 to form a therapeutic current path via the electrode poles 311, 312.

The switches S1-S7 may be controlled by an electronic control circuitry, which is operated by the energy as received from the external energy transmitting device (in the embodiment of Fig. 1 the implantable cardioverter defibrillator device 1).

In the embodiment of Fig. 10, the signal generation circuit arrangement 323 comprises a current limiting component 324, for example in the shape of a current limiting diode or a Zener diode, which serves to limit a current and/or voltage of an electrical stimulation pulse as output via the H bridge formed by the switches S4 to S7. By means of the current limiting component 324 it can be avoided that an output signal comprises an excessive voltage level and/or current beyond a defined maximum. For example, using the current limiting component 324 an output voltage and current may be limited to a value equal to or below 50 V respectively equal to or below 100 mA.

The idea underlying the invention is not limited to the embodiments described above, but may be implemented in an entirely different fashion.

The implantable medical device may be used in combination with an implantable cardioverter defibrillator device or with another device separate from the implantable medical device. The separate device functions as an energy transmitting device to transmit an energy signal to the implantable medical device, such that energy is supplied to the implantable medical device from the energy transmitting device. The energy transmitting device may be implanted or may be external to the patient.

### List of reference numerals

- 1: Non-transvenous implantable cardioverter defibrillator device
- 10: Generator device
- 100: Housing
- 101: Connection block
- 102: Processing circuitry
- 103: Shock generation circuitry
- 104: Energy storage (battery)
- 11: Electrode lead
- 110: Lead body
- 111: Proximal end
- 112: Distal end
- 113, 114: Electrode pole
- 115: Shock electrode (coil)
- 2: External device
- 3: Implantable medical device
- 30: Body
- 31: Electrode pole arrangement
- 310-313: Electrode pole
- 32: Processing circuitry
- 320: Filter unit
- 321: Demodulating circuit arrangement
- 322: Energy storage circuit arrangement
- 323: Signal generation circuit arrangement
- 324: Current limiting device
- 33: Fixation device
- C1-C4: Capacitor
- CS: Carrier signal
- D1-D4: Diode
- H: Heart
- L: Longitudinal axis
- PWᵢ₋₁, PWᵢ: Pulse width
- R1, R2: Resistor
- RPᵢ: Repetition interval
- RS: Received signal
- S1-S7: Switch
- S_{i-1,trans}, S_{i,trans}: Transmit pulses
- S_{i-1,rec}, S_{i,rec}: Energy pulses
- T1, T2: Time point
- TS: Energy signal
- V1: Voltage source

## Claims

1. An implantable medical device (3) for emitting an electrical stimulation signal to perform a therapeutic action, comprising:
a processing circuitry (32) for generating said electrical stimulation signal, and
an electrode pole arrangement (31) comprising a multiplicity of electrode poles (310-313) configured to emit said electrical stimulation signal to perform said therapeutic action;
wherein the processing circuitry (32) is configured to extract electrical energy from an energy signal (TS) received, using a first pair of electrode poles of said multiplicity of electrode poles (310-313), from an energy transmitting device (1) external to the implantable medical device (3) to obtain extracted electrical energy and to produce said electrical stimulation signal using said extracted electrical energy for emission by a second pair of electrode poles of said multiplicity of electrode poles (310-313).

2. The implantable medical device (3) according to claim 1, **characterized in that** the first pair of electrode poles and the second pair of electrode poles have at least one electrode pole in common.

3. The implantable medical device (3) according to claim 1 or 2, **characterized by** a body (30) on which said multiplicity of electrode poles (310-313) are arranged.

4. The implantable medical device (3) according to claim 3, **characterized in that** the body (30) is made from an electrically insulating material or at least comprises an electrically non-conductive outer surface.

5. The implantable medical device (3) according to claim 3 or 4, **characterized in that** the body (30) is flexibly bendable.

6. The implantable medical device (3) according to one of claims 3 to 5, **characterized in that** the body (30) has an elongated shape generally extending along a longitudinal axis (L), wherein at least some of said multiplicity of electrode poles (310-313) are displaced with respect to one another along the longitudinal axis (L).

7. The implantable medical device (3) according to one of claims 3 to 6, **characterized in that** at least some of said multiplicity of electrode poles (310-313) are formed as ring electrodes arranged on said body (30).

8. The implantable medical device (3) according to one of the preceding claims, **characterized in that** the energy signal (TS) is formed using a carrier signal (CS) in a pre-defined frequency range above 10 kHz, preferably above 30 kHz.

9. The implantable medical device (3) according to claim 8, **characterized in that** the energy signal (TS) comprises a multiplicity of pulses (S_{i-1,trans}, S_{i,trans}) formed by modulating said carrier signal (CS).

10. The implantable medical device (3) according to one of the preceding claims, **characterized in that** the processing circuitry (32) comprises a filter unit (320) for performing a frequency filtering of said energy signal.

11. The implantable medical device (3) according to one of the preceding claims, **characterized in that** the processing circuitry (32) comprises a demodulating circuit arrangement (321) for demodulating said energy signal (TS) to obtain said extracted electrical energy, the demodulating circuit arrangement (321) comprising at least one diode (D1-D4).

12. The implantable medical device (3) according to one of the preceding claims, **characterized in that** the processing circuitry (32) comprises an energy storage circuit arrangement (322) for storing said extracted electrical energy, the energy storage circuit arrangement (322) comprising at least one capacitor (C1-C4).

13. The implantable medical device (3) according to one of the preceding claims, **characterized in that** the processing circuitry (32) comprises a signal generation circuit arrangement (323) for producing said electrical stimulation signal from said extracted electrical energy.

14. The implantable medical device (3) according to claim 13, **characterized in that** said signal generation circuit arrangement (323) comprises at least one current limiting device (324) for limiting a current for producing said electrical stimulation signal.

15. A method for operating an implantable medical device (3) for emitting an electrical stimulation signal to perform a therapeutic action, the method comprising:
generating, using a processing circuitry (32), said electrical stimulation signal; and
emitting, using an electrode pole arrangement (32) comprising a multiplicity of electrode poles (310-313), said electrical stimulation signal to perform said therapeutic action;
wherein the processing circuitry (32), for generating said electrical stimulation signal, extracts electrical energy from an energy signal (TS) received, using a first pair of electrode poles of said multiplicity of electrode poles (310-313), from an energy transmitting device (1) external to the implantable medical device (3) to obtain extracted electrical energy and produces said electrical stimulation signal using said extracted electrical energy for emission by a second pair of electrode poles of said multiplicity of electrode poles (310-313).
